(19) **Europäisches Patentamt**
**European Patent Office**
**Office européen des brevets**

(11) **EP 1 568 357 B1**

(12) **EUROPEAN PATENT SPECIFICATION**

(45) Date of publication and mention
of the grant of the patent:
**30.11.2016 Bulletin 2016/48**

(51) Int Cl.:
*A61K 8/02* *(2006.01)* *A61K 8/86* *(2006.01)*
*A61K 8/37* *(2006.01)* *A61K 8/89* *(2006.01)*
*A61Q 19/10* *(2006.01)* *A61K 8/891* *(2006.01)*
*A61K 8/58* *(2006.01)* *A61K 8/31* *(2006.01)*
*A61Q 1/14* *(2006.01)* *A61K 8/39* *(2006.01)*

(21) Application number: **04024932.8**

(22) Date of filing: **20.10.2004**

(54) **Cleansing composition and cleansing sheet**

Abschminkmittel und Reinigungstuch

Composition démaquillante et feuille de nettoyage

(84) Designated Contracting States:
**DE FR GB**

(30) Priority: **24.02.2004 JP 2004047504**

(43) Date of publication of application:
**31.08.2005 Bulletin 2005/35**

(73) Proprietor: **KAO CORPORATION**
**Tokyo 103-8210 (JP)**

(72) Inventors:
• **Ueda, Atsuhiro**
**Sumida-ku, Tokyo 131-8501 (JP)**
• **Tsuda, Hiroko**
**Sumida-ku, Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
WO-A-95/03781     US-A- 5 770 554
US-A- 5 840 676     US-A- 6 063 397

Remarks:
The file contains technical information submitted after
the application was filed and not included in this
specification

**Description**

Field of the Invention

The present invention relates to a cleansing composition and a cleansing sheet for removing makeup cosmetics.

Background of the invention

For removing makeup, some techniques are known, such as use of a surfactant aqueous solution and use of an oil in the form of a cleansing gel, cream and oil or the like. Mascara, which is relatively difficult to remove among makeup cosmetics, includes aqueous mascara containing a water-soluble polymer for a curl retaining effect and oily mascara which is relatively proof against sweat and water. Although the conventional surfactant aqueous solution can quickly remove the aqueous mascara which is easily dissolved or dispersed in water, it cannot remove the oily mascara. On the other hand, although the oil-based cleansing agent can remove the oily mascara, it cannot remove the aqueous mascara.

According to the recent popularity of mascara, a film forming type mascara which utilizes a film forming ability of latex polymer, in particular, the mascara which contains an alkyl acrylate copolymer having an excellent film forming ability is widely used in both oily type and aqueous type of mascara. Since the latex polymer has a water-insoluble polymer present in a state of emulsion in water to form a film by generating strong bond between the polymer particles according to the water evaporation, the latex polymer in the form of a film can neither be easily dispersed in water or the surfactant aqueous solution nor be quickly dissolved in oil. Therefore, a film once formed cannot be removed effectively by the conventional makeup removing technique using the surfactant aqueous solution or the oil agent.

As a technique for removing oily and film forming eye makeup cosmetics, JP-A No. 5(1993) - 163116 (the term "JP-A" as used herein means an "unexamined published Japanese Patent Application") discloses an eye makeup removing agent having an isoparaffin emulsified in water by a polyoxyethylene alkyl ether-based surfactant. However, it has an insufficient cleansing performance on the mascara containing a large amount of an alkyl acrylate copolymer capable of forming a strong film.

Additionally, as a technique for removing the makeup without using a common surfactant, JP-A No. 11(1999)-503461 discloses a technique of coexisting a non-emulsifying surfactant in an emulsion containing an acrylic acid-alkyl acrylate copolymer based emulsifying agent, an oil and water. JP-A No. 9(1997)-48706 discloses a cleansing composition containing an oil and water with a large amount of a polyol as a humectant. Moreover, JP-A No. 62 (1987) -135406 discloses a makeup removing agent containing a low boiling point isoparaffin or a low boiling point silicone oil dispersed in the aqueous phase by a polyacrylic thickening agent. JP-A No. 9(1997)-110638 discloses a makeup removing agent containing a water-soluble polysaccharide, a low boiling point oil agent and water as essential components. However, they can remove the oily or aqueous mascara but cannot effectively remove the film forming type mascara.

Moreover, JP-A No. 2(1990) - 104511 discloses a composition for cleansing tissue containing a polyalkylene glycol, ethanol, a water-soluble polyhydric alcohol, a surfactant and water. JP-A No. 9 (1997) - 143028 discloses a composition for removing makeup cosmetics containing one or more glucosides selected from a polyoxyethylene methyl glucoside and a polyoxypropylene methyl glucoside, and a nonionic surfactant having HLB value of 10 or more. However, although they can remove the aqueous type mascara, they cannot remove the oily mascara nor effectively remove the film forming type mascara.

On the other hand, in terms of a cleansing sheet, the following prior art has been proposed. JP-A No. 2001-302450 discloses a cleansing material formed by a sheet impregnated with an emulsion cosmetic that has a viscosity of 200 to 4,000 mPa·s. Furthermore, JP-A No. 2003-335627 discloses a sheet impregnated with a two-phase (O/W) type cosmetic having thixotropic properties. These sheets can remove the oily type mascara and the aqueous type mascara but cannot effectively remove the film forming type mascara.

WO 95/03781 is a document relating to personal cleansing compositions comprising mineral oil and compounds having a polyoxypropylene structure.

SUMMARY OF THE INVENTION

The present invention provides a cleansing composition comprising (A) an aqueous phase containing about 1 to 25% by weight, in the aqueous phase, of a water-soluble compound (a) which has a polyoxypropylene structure having 3 or more addition number of propylene oxide but neither an alkyl group having 4 or more carbon atoms nor a polyoxyethylene structure and (B) an oil phase which is liquid at 30°C, wherein the weight ratio (A) : (B) of the aqueous phase (A) and the oil phase (B) is about 97:3 to 40:60, and more than about 10% by weight of water based on the total composition.

Furthermore, the present invention provides a cleansing sheet comprising a nonwoven fabric which comprises a fiber having a cellulose content of at least 70% by weight and has a density of about 0.15 to 0.3 g/cm$^3$ impregnated

with an oil-in-water emulsion composition comprising (A) an aqueous phase containing about 1 to 25% by weight, in the aqueous phase, of a water-soluble compound (a) which has a polyoxypropylene structure having 3 or more addition number of propylene oxide but neither an alkyl group having 4 or more carbon atoms nor a polyoxyethylene structure, (B) an oil phase containing about 50% by weight or more of an oil (b) having a boiling point of about 160 to 300°C at 1,013.25 hPa, and (C) about 0.01 to 1% by weight of an aqueous thickening agent having a hydrophobic part, wherein the weight ratio (A) : (B) of the aqueous phase (A) and the oil phase (B) is about 97:3 to 40:60.

DETAILED DESCRIPTION OF THE INVENTION

First, a cleansing composition of the present invention and an oil-in-water emulsion composition used for a cleansing sheet of the present invention are explained.

Component (A) used in the present invention is an aqueous phase containing about 1 to 25% by weight, in the aqueous phase, of a water-soluble compound (a) (hereinafter, it may be referred to as a "compound (a)") which has a polyoxypropylene structure having 3 or more addition number of propylene oxide but neither an alkyl group having 4 or more carbon atoms nor a polyoxyethylene structure in the aqueous phase. The aqueous phase (A) has little cleansing performance on a common oily makeup cosmetic but has a certain level of cleansing performance on a polymer film. By combining the aqueous phase (A) and oil phase (B) which has excellent cleansing performance on oily makeup cosmetics and also some cleansing performance on polymer film at a specific ratio, a high cleansing performance on oily type mascara and other common makeup cosmetics can be provided. Furthermore, the removing effect on a film forming type mascara, in particular, on a mascara containing a large amount of an alkyl acrylate copolymer capable of forming a strong film, can be remarkably improved.

Compound (a) contained in aqueous phase (A) has a polyoxypropylene structure having 3 or more addition number of propylene oxide from the viewpoint of the cleansing performance on the polymer film. Since the compound (a) does not have an alkyl group having 4 or more carbon atoms, it does not behave like a surfactant, and thus it can hardly form a micelle in water and can easily enter into a fine part of the polymer film.

Furthermore, it is assumed that the polyoxypropylene structure acts on a strong bond between the polymer molecules so as to promote re-dispersion of the polymer film into water, thus obtaining an excellent removing effect for film forming type mascara. However, if the compound (a) has a polyoxyethylene structure, its distribution to the aqueous phase is promoted so as to weaken the cleansing effect.

Moreover, the compound (a) has adequate water solubility. The water solubility here denotes a solubility of 1% by weight or more to water at 25°C.

Compound (a) contained in the aqueous phase (A) of the present invention is preferably a compound having a hydrophilic group such as a hydroxyl group, a glyceryl group, a polyglyceryl group and a sugar residue bonded with the polyoxypropylene structure of the 3 or more addition number of propylene oxide.

Specific examples of the compound (a) include polyoxypropylene diglyceryl ether, polyoxypropylene glyceryl ether, polyoxypropylene methyl glucoside, polypropylene glycol or the like.

The polyoxypropylene diglyceryl ether is produced by addition polymerization of propylene oxide to diglycerin. Among them, addition number of propylene oxide is preferably 4 to 24 moles from the viewpoint of the cleansing performance on the polymer film, and more preferably 6 to 18 moles. The polyoxypropylene glyceryl ether is produced by addition polymerization of propylene oxide to glycerin. Among them, addition number of propylene oxide is preferably 8 to 24 moles from the viewpoint of the cleansing performance on the polymer film, and more preferably 8 to 16 moles. The polyoxypropylene methyl glucoside is produced by addition polymerization of propylene oxide tomethyl glucoside. Among them, addition number of propylene oxide is preferably 5 to 25 moles from the viewpoint of the cleansing performance on the polymer film, and more preferably 10 to 20 moles. As to the polypropylene glycol, addition number of propylene oxide is preferably 5 to 20 moles from the viewpoint of the cleansing performance on the polymer film, and more preferably 7 to 16 moles.

In particular, polyoxypropylene diglyceryl ether and polyoxypropylene glyceryl ether have effects of improving compatibility of the composition with the skin and provide a moist feeling without stickiness after use. The polyoxypropylene diglyceryl ether is even more preferable.

The compound (a) contained in the aqueous phase (A) can be used solely or in combination of two or more kinds. The preferred content of the compound (a) in the aqueous phase (A) is 1% by weight or more based on the total amount of the aqueous phase (A) in terms of obtaining a sufficient cleansing performance on the film forming type mascara, and 25% by weight or less so as not to cause a residual feeling to the skin after use. In terms of the balance of the cleansing performance and the feeling upon use, it is more preferably 3 to 20% by weight based on the total amount of the aqueous phase (A) and even more preferably 5 to 15% by weight.

The content of water contained in the aqueous phase (A) is preferably 10% by weight or more based on the total amount of the composition, and more preferably 30% by weight or more, and even more preferably 50% by weight or more in terms of a sufficient cleansing performance and a good use feeling.

Aqueous phase (A) contains the above-mentioned compound (a) and water as main components, and it may contain other water-soluble components. For example, alcohols such as ethanol and a polyol improve the feeling upon use. In particular, addition of ethanol provides an effect of improving a fresh feeling, and addition of 1,3-butylene glycol or glycerin provides an excellent effect of improving a moist feeling.

Next, component (B) used in the present invention is an oil phase which is in a liquid state at 30°C, and preferably has an excellent cleansing performance on oily type mascara and common oily makeup cosmetics. Moreover, component (B) has a dissolving ability with respect to the polymer film, although it is not as strong as component (A), and thereby having some cleansing performance on the polymer film. An oil phase having a low viscosity has a high permeability into fine parts and a high solubility of stains, thus obtaining an excellent cleansing performance. Furthermore, it has a good feeling upon use without a strong oily feeling.

Therefore, it is preferable that oil phase (B) has a viscosity of 10 mPa·s or less at 30°C. As the oil phase (B), oily components may be used solely or in combination of two or more kinds. When two or more oily components are used in combination, those components being not in a liquid state at 30°C or having viscosity of more than 10 mPa·s at 30°C are also used, provided that the mixture containing such an oily component has a viscosity of 10 mPa·s or less at 30°C. Here, the viscosity can be measured by a BM type viscometer (for example, one produced by TOKIMEC Inc.) by using Rotor No. 1, at 60 rpm.

Specific examples of the oily component having a viscosity of 10 mPa·s or less at 30°C include liquid isoparaffin, alkyl 1,3-dimethyl butyl ether (JP-A No. 9(1997)-87223), cetyl 2-ethylhexanate, isopropyl myristate, isopropyl palmitate, isononyl isononanoate, isotridecyl isononanoate, diisobutyl adipate, diisopropyl adipate, octamethyl trisiloxane, decamethyl cyclopentasiloxane, dodecamethylpentasiloxane, and octamethyl cyclotetrasiloxane.

In particular, the oily component having a molecular weight of 300 or less is more preferable in terms of high cleansing performance and an excellent feeling upon use without an oily feeling after use. Specifically, light liquid isoparaffin, isopropyl myristate, isopropyl palmitate, isononylisononanoate, diisobutyl adipate, diisopropyl adipate, octamethyl trisiloxane, octamethyl cyclotetrasiloxane, or the like may be used.

Moreover, oil (b) having a boiling point of 160 to 300°C at 1,013.25 hPa has a low viscosity and a high permeability into the fine parts, thus obtaining a high dissolving or dispersion property of the makeup cosmetics. Furthermore, since the low boiling point oil has little residual feeling after use without a strong oily feeling, it is preferably impregnated into a sheet material and used by wiping off the makeup from the skin. In this case, the content of the above-mentioned oil (b) is preferably 50% by weight or more and more preferably 70% by weight or more based on the total amount of the oil phase (B) in terms of the cleansing performance on the oily makeup cosmetics and the feeling upon use with little oily feeling.

Among them, an oil having a boiling point of 160 to 250°C at 1,013.25 hPa is preferable for a less residual feeling on the skin after use. Furthermore, an oil having a boiling point of 160 to 230°C at 1,013.25 hPa is more preferable, and an oil having a boiling point of 160 to 210°C at 1,013.25 hPa is even more preferable in terms of the cleansing performance and the absence of a residual oily feeling on the skin.

The above-mentioned oil is selected from light liquid isoparaffin, and volatile silicone. In terms of the cleansing performance, it is more preferable to use the light liquid isoparaffin. They are used solely or in combination of two or more kinds.

The light liquid isoparaffin is a mixture of a branched hydrocarbon having 8 to 18 carbon atoms in general and has a distinctive odor by including low molecular weight hydrocarbons. Since such an odor may not be acceptable by a user, it is preferable that a low molecular weight component having 8 to 9 carbon atoms are contained as little as possible. On the other hand, when a hydrocarbon having 16 to 18 carbon atoms is included in large amounts, it tends to deteriorate the cleansing performance and to have a strong oily feeling after use. In terms of these points, it is preferable that an isoparaffin-based hydrocarbon having 10 to 15 carbon atoms is included in large amounts. Among them, in terms of the balance of the odor, the use feeling and the cleansing performance, it is preferable that isododecane having 12 carbon atoms is included in large amounts. In particular, an oil containing 90% by weight or more, more preferably 95% by weight or more of isododecane, can be used preferably in the present invention.

The trade names of the light liquid isoparaffin include MARCASOL R (Maruzen Petrochemical Co., Ltd.), IP SOLVENT 1620, IP SOLVENT 2028 (Idemitsu Petrochemical Co., Ltd.), ISOPER L, ISOPER H (Exxon Chemical Ltd.), ISOSOL 300, ISOSOL 400 (Nippon Petrochemical Company, Limited). In terms of containing isododecane of a high degree of purity, the MARCASOL R (Maruzen Petrochemical Co., Ltd.) may be used even more preferably.

Moreover, examples of the volatile silicone oil include linear dimethyl polysiloxanes with a low degree of polymerization such as octamethyl trisiloxane (boiling point 153°C) and dodecamethyl pentasiloxane (boiling point 229°C), cyclic silicone oils such as octamethyl cyclotetrasiloxane (boiling point 172°C) and decamethyl cyclopentasiloxane (boiling point 205°C).

In terms of a sufficient cleansing performance and a good feeling upon use without a strong oily feeling, the weight ratio (A) : (B) of aqueous phase (A) and oil phase (B) is 97:3 to 40:60, preferably 90:10 to 70:30, and more preferably 88:12 to 75:25.

Although a mixture of aqueous phase (A) and oil phase (B) may be separated into two-phases after settling, it can be temporarily homogenized by shaking and mixing, and then used in such manner that a cosmetic cotton is impregnated with it and employed for wiping off the makeup from the skin, or that it is taken directly on the user's hand and spread on the makeup. Furthermore, a cleansing sheet can also be provided by impregnating a sheet material made of a nonwoven fabric or the like with the mixture.

In the case that the mixture of aqueous phase (A) and oil phase (B) is liable to separate into two-phases in a short time, the cleansing composition of the present invention may further contain an aqueous thickening agent as component (C) for the purpose of facilitating the use. By containing the aqueous thickening agent, the homogeneous state can be retained at the time of shaking and mixing the composition and the viscosity is raised so as to facilitate the use, thereby improving comfort to the skin and massage performance.

Specific examples of the aqueous thickening agents include the following water-soluble polymers. Examples of the natural water-soluble polymers include plant-based polymers such as gum Arabic, gum tragacanth, galactan, gum guaiac, carob gum, karaya gum, carrageenan, pectin, agar, quince seed gum, brown algae extract, starch (rice, corn, potato, and wheat), and glycyrrhizinic acid; bacteria-based polymers such as xanthan gum, dextrin, succino glucan and pullulan; and animal-based polymers such as collagen, casein, albumin and gelatin. Examples of the semi synthetic water-soluble polymers include starch-based polymers such as carboxy methyl starch and methyl hydroxyl propyl starch; cellulose-based polymers such as methyl cellulose, nitro cellulose, ethyl cellulose, methyl hydroxyl propyl cellulose, hydroxyl ethyl cellulose, cellulose sodium sulfate, hydroxyl propyl cellulose and sodium carboxy methyl cellulose (CMC) ; and alginic acid-based polymers such as sodium alginate and propylene glycol alginate. Examples of the synthetic water-soluble polymers include vinyl polymers such as polyvinyl alcohol, polyvinyl methyl ether, polyvinyl pyrrolidone, carboxy vinyl polymer (CARBOPOL 940, 941: Noveon, Inc.); polyoxyethylene-based polymers such as polyethylene glycol 20000, polyethylene glycol 6000 and polyethylene glycol 4000; copolymers such as polyoxyethylene polyoxypropylene copolymer; acrylic-based polymers such as sodium polyacrylate, polyethyl acrylate and polyacryl amide; polyethylene imine; and cationic polymer.

Furthermore, by using an aqueous thickening agent having a hydrophobic part, aqueous phase (A) is thickened and oil phase (B) is dispersed in aqueous phase (A) in oil-in-water form, thus obtaining a composition having a uniform appearance. The aqueous thickening agent having a hydrophobic part here denotes a water-soluble polymer having a hydrophobic group with 10 or more carbon atoms as the side chain.

As to the composition used for the cleansing sheet of the present invention, in order to prevent change of the composition in the sheet material and consequently to obtain the cleansing material storage stability and good cleansing performance, the aqueous thickening agent having a hydrophobic part is preferably used as component (C) for preparing an oil-in-water emulsion composition, and impregnating the sheet material therewith.

In the case of using the aqueous thickening agent having a hydrophobic part, the interaction between the hydrophobic part and the oil allows the oil to be sustained in water relatively stably. However, it is not so finely emulsified as in the case of emulsification using an ordinary surfactant, and thus the oil can easily be released at the time of use without deterioration of the cleansing performance. In other words, according to the use of such an aqueous thickening agent, the oil in the composition can be dispersed to the degree not to deteriorate the cleansing performance so that the homogeneity and the stability of the liquid can be improved to provide the stable performance as the cleansing sheet. It is presumed that after impregnation of the sheet material with the composition the aqueous thickening polymer is entangled between the fibers together with the oil to retain the oil stably in the sheet material.

Examples of the aqueous thickening agent having a hydrophobic part include an acrylic acid-·alkyl(C10 to C30)methacrylate copolymer (produced by Noveon, Inc., trade name: PEMULEN TR-1, PEMULEN TR-2, CARBOPOL ETD, or the like), hydroxyl ethyl cellulose hydroxyl propyl stearyl ether hydroxyl propyl sodium sulfonate (JP-A No. 9(1997)-235301).

The above-mentioned aqueous thickening agents (C) can be used solely or in combination of two or more kinds. When using in the cleansing composition of the present invention, the content of aqueous thickening agent (C) is preferably 0.01 to 5% by weight based on the total amount of the composition and more preferably 0.01 to 2% by weight and even more preferably 0.01 to 1% by weight in terms of easiness of handling, spreadability on the skin and prevention of sliminess. In particular, when using in the oil-in-water emulsion composition for impregnating the cleansing sheet of the present invention, the content of the above-mentioned aqueous thickening agent having a hydrophobic part is preferably 0.01% by weight or more based on the total amount of the composition in terms of the emulsion stability of the emulsion composition, and preferably 1% by weight or less based on the total amount of the composition in terms of providing a good processing property by a viscosity for easily impregnating the sheet material. Among them, in terms of the balance between the emulsion stabilizing ability and the processing property, it is preferably in the range of 0.1 to 0.5% by weight based on the total amount of the composition.

If necessary, a neutralizing agent can be used for neutralizing the above-mentioned aqueous thickening agents to obtain suitable thickening. Example of the neutralizing agent include an inorganic alkaline agent such as sodium hydroxide and potassium hydroxide, a basic amino acid such as L-alginic acid, amines such as triethanol amine, and ammonia. The amount of the neutralizing agent may be determined according to the kind and the amount of the aqueous thickening agent used, and it is preferably about 0.005 to 0.5% by weight based on the total amount of the composition. The neutralizing agent may be added as an aqueous solution or directly mixed with the aqueous thickening agent.

Since an emulsion system using a surfactant is not sufficient in the makeup removing performance due to stable dispersion of the oil in the emulsion system, it is preferable that the function of stabilizing the emulsion or dispersion is provided by the above-mentioned aqueous thickening agent. Therefore, in the present invention, surfactants may be used in a small amount so as not to deteriorate the effect of the present invention, for the purpose of improving the comfort to the skin of the composition or for the purpose of improving the wettability or the permeating property of the composition with respect to the sheet material.

In order to use the surfactant in the range not to deteriorate the cleansing performance of aqueous phase (A) and oil phase (B), the content of the surfactant is preferably 2% by weight or less based on the total amount of the composition and more preferably 0.5% by weight or less and even more preferably less than 0.05% by weight.

The surfactant can be used solely or in combination of two or more kinds. In terms of the mildness to the skin, specific examples include polyoxyethylene alkyl ethers, sucrose fatty acid esters, fatty acid monoglycerides, sorbitan fatty acid esters optionally having polyoxyalkylene chains added thereto, polyoxyethylene hardened castor oil and fatty acid esters thereof, and polyoxyalkylene fatty acid esters.

The composition of the present invention may further contain various additives such as a humectant, an oil gelling agent, an ultraviolet absorber, a perfume, a pigment, a dye, salts, a stabilizing agent, a preservative, an antioxidant, vitamins, a protein, and a conditioning agent, in a suitable amount so long as the objects and the effects of the present invention are not deteriorated thereby.

The cleansing composition of the present invention can be produced according to an ordinary method using the above-mentioned materials, alternatively in the form of a liquid, a gel, a cream or a two-phase separated type.

As a method for removing the makeup using the cleansing composition of the present invention, there may be a method of impregnating a wiping medium with the composition directly or after shaking and homogenizing it, followed by wiping the makeup off the skin, and a method of taking the composition on the hand and applying the same to the skin, followed by wiping off with a wiping medium or by washing off. Here, examples of the wiping medium include a cotton and a tissue paper.

The cleansing composition of the present invention can remove effectively not only ordinary makeup but also the hard to remove mascara. It can obtain excellent cleansing performance on both oily type mascara and the film forming type mascara containing a large amount of an alkyl acrylate copolymer capable of forming a strong film. The cleansing composition according to the present invention can effectively remove all kinds of makeup and provide an excellent feeling upon use.

Next, the cleansing sheet of the present invention is explained.

Among the above-mentioned cleansing composition, a composition comprising (A) an aqueous phase containing about 1 to 25% by weight, in the aqueous phase, of a water-soluble compound (a) which has a polyoxypropylene structure having 3 or more addition number of propylene oxide but neither an alkyl group having 4 or more carbon atoms nor a polyoxyethylene structure, (B) an oil phase containing about 50% by weight or more of an oil (b) having a boiling point of about 160 to 300°C at 1,013.25 hPa and (C) about 0.01 to 1% by weight of an aqueous thickening agent having a hydrophobic part, wherein the weight ratio (A) : (B) of aqueous phase (A) and oil phase (B) is about 97:3 to 40:60 is particularly preferable as an oil-in-water emulsion composition for impregnating a sheet like base material therewith in terms of the excellent cleansing performance and the excellent feeling upon use.

The cleansing sheet of the present invention includes a specific nonwoven fabric impregnated with the above-mentioned oil-in-water emulsion composition. Since the cleansing material of the present invention is sheet like, it can be used immediately after taking out from a package and the makeup can be conveniently and quickly removed only by wiping off.

The viscosity of the oil-in-water emulsion composition used for the cleansing sheet is preferably 2,000 mPa·s or less in terms of the impregnation efficiency in processing, and more preferably 300 to 800 mPa·s in terms of ease of seeping of the liquid from the sheet material in use. Here, the viscosity denotes the value measured by a BM type viscometer (e. g. produced. by TOKIMEC Inc.) under the conditions of Rotor No. 2, 6 rpm, and 30°C.

Moreover, the oil-in-water emulsion composition to be used for the cleansing sheet may separate into a transparent aqueous phase and an oil-in-water emulsion phase after settling. A relatively unstable emulsion composition is preferable in terms of the cleansing performance and the feeling upon use. For example, after prepared homogeneously, an emulsion may separate into an aqueous phase and emulsion phase during the time between one day and one month when it is left still at 30°C. In this case, the impregnation of the nonwoven fabric is preferably executed before progress of said separation. By using such a relatively unstable emulsion composition, both the favorable seeping of the liquid from the sheet and the excellent cleansing performance can be provided compared with a stable emulsion composition with the aqueous phase also thickened by the polymer compound.

The nonwoven fabric as the sheet base material for the cleansing sheet of the present invention is preferably made of a fiber having a cellulose content of about 70% or more by weight and has a density of about 0.15 to 0.3 g/cm$^3$ in terms of having a good feeling of the skin upon use and stably maintaining the quality during long term storage in a stacked state. According to the impregnation of such a hydrophilic nonwoven fabric having a relatively high density, movement of the emulsion composition in the nonwoven fabric can be restrained, thus maintaining the amount and the composition of the emulsion composition in the nonwoven fabric. Namely, even in the case of using a slightly unstable emulsion composition, the nonwoven fabric sustains the aqueous phase. Furthermore, the aqueous thickening polymer containing the oil is entangled between the fibers so as to stably support the oil in the nonwoven fabric.

In order to restrain the movement of the oil-in-water emulsion composition impregnated in the sheet base material, it is preferable that the nonwoven fabric is hydrophilic for stably supporting the aqueous phase as the outer phase. In other words, as the nonwoven fabric, those made of a fiber having a cellulose content of at least about 70% by weight are used. In terms of stably supporting the aqueous phase, the cellulose content is more preferably 80% by weight or more.

Specific examples of the cellulose fibers include cotton, rayon or the like. In particular, cotton is preferable in terms of providing the feeling of certainly wiping off the makeup without slipping during use. Moreover, the nonwoven fabric preferably is obtained by the hydroentangling method in terms of providing a smooth favorable texture to the skin.

As far as satisfying the above-mentioned benefits, a hydrophobic fiber can be mixed for modification of the sheet property. Examples of the hydrophobic fiber include a polyester-based fiber and a polyolefin-based fiber. Examples include polyethylene terephthalate (PET), polybutylene terephthalate (PBT), polypropylene (PP) and polyethylene (PE).

Moreover, in order to sustain stably the impregnated oil-in-water emulsion composition and restrain the vertical movement of the liquid in the stacked sheet products, it is preferable that the nonwoven fabric has a relatively high density. The density of the nonwoven fabric is preferably 0.15 g/cm$^3$ or more so as not to easily move the liquid to the down side of the stacked sheets by gravity. On the other hand, the preferred density of the nonwoven fabric is 0.3 g/cm$^3$ or less in terms of facilitating impregnation of the sheet with the liquid and providing a soft feeling of the sheet upon use. In terms of the balance of the restraint of the movement of the liquid and the feeling upon use, the density of the nonwoven fabric is more preferably 0.17 to 0.25 g/cm$^3$.

Moreover, the average basis weight (weight per unit area) of the nonwoven fabric is preferably 40 g/m$^2$ or more in terms of providing easy handling without shrinkage at the time of wiping and preventing the stains from transferring to the rear side. It is also preferably 100 g/m$^2$ or less in terms of obtaining a flexible feeling without bulkiness at the time of stacking. In particular, the average basis weight is more preferably 50 to 80 g/m$^2$ in terms of providing easy handling in use and an appropriate bulkiness at the time of stacking.

The nonwoven fabric may be a sheet material prepared by laminating or bonding two or more kinds of nonwoven fabric. Moreover, the nonwoven fabric useful herein has a suitable shape, size and thickness from the standpoint of handling properties. For example, the nonwoven fabric is preferably in a square, rectangular, circular, or oval shape with a side or diameter of about 5 to 20 cm and a thickness of about 0.2 to 0.5 mm.

The nonwoven fabric preferably is impregnated with the oil-in-water emulsion composition in an amount of from 2 to 5 g , and more preferably from 2. 5 to 4.5 g; per 1 g of the nonwoven fabric in terms of a good feeling upon use.

The cleansing sheet of the present inventionmay be produced by impregnating a nonwoven fabric with an emulsion composition in such manner that the emulsion is applied on the nonwoven fabric by dropping or flowing from a nozzle, or is sprayed on the nonwoven fabric, and then the sheet is left as it is so as to allow the emulsion to sufficiently permeate into the sheet material.

The cleansing sheet of the present invention is packed one by one or with several sheets into a bag or other package and then sealed. Before using, the package is opened and a cleansing sheet is taken out. Then, the cleansing sheet is applied onto the skin so that the makeup cosmetics are wiped off.

Thus, the cleansing sheet of the present invention can remove conveniently and quickly not only ordinary makeup such as foundation and lipstick, but also aqueous mascara and oily mascara, furthermore, even a film forming typemascara containing a large amount of an alkyl acrylate copolymer. The cleansing sheet of the present invention has a favorable texture to the skin during and after use, and an excellent stability in a product form in which the sheets are stacked.

EXAMPLE

By reference to the following Examples, the present invention is further explained and the embodiments of the present invention are illustrated.

Example A series: cleansing composition

(Evaluation method of cleansing performance)

For the evaluation of cleansing performance, an oily mascara and a film forming type aqueous mascara containing a large amount of an alkyl acrylate copolymer were used.

A predetermined amount of the mascara was applied on a glass slide evenly to form a circle of 1.2 cm in diameter and dried by allowing to stand for 12 hours. 0.0070 g of TESTIMO MASCARA EFFECT CURLLONG BK18 (trade name) produced by Kanebo, Ltd. as the film forming type aqueous mascara and 0.0045 g of SPORTS BEAUTY FASIO POWER STAY MASCARA CURLLONG BK001 (trade name) produced by KOSÉ Corporation as the oily mascara were used. Because of the difference in the liquid property of each mascara, the amount of coating of each mascara is different.

Since the cleansing composition having components (A) and (B) separates quickly even after mixing, at the time of the cleansing performance evaluation, cotton was impregnated with 3.8 g of the cleansing composition per 1 g of the cotton and then lightly rubbed so as to allow the composition to permeate into the entire cotton uniformly. Thereafter, the cotton was placed on the mascara and lightly pressed thereon for 5 seconds. Next, the mascara was wiped off with a constant pressure ($100 \text{ g/cm}^2$) to count the number of the wiping action necessary to remove the mascara. And then, the cleansing performance on each of the mascara was evaluated according to the following criterion (Example A1, Comparative Examples A1 to A5) .

<Criterion for evaluation of the cleansing performance on the film forming type aqueous mascara>

| | |
|---|---|
| ◎ | 14 times or less |
| ○ | 15 to 19 times |
| ○Δ | 20 to 24 times |
| Δ | 25 to 29 times |
| × | 30 times or more |

<Criterion for evaluation of the cleansing performance on the oily mascara>

| | |
|---|---|
| ◎ | 5 times or less |
| ○ | 6 to 10 times |
| ○Δ | 11 to 15 times |
| Δ | 16 to 20 times |
| × | 21 times or more |

Similar evaluation was carried out for the liquid type cleansing composition homogenized by using component (C). 0.1 g of the cleansing composition was applied onto the mascara on the glass slide. After massaging with a finger for removing the stains 60 times with respect to the film forming type aqueous mascara and 30 times with respect to the oily mascara, they were wiped off with tissue paper. And then, they were evaluated according to the following criterion (Examples A2 to A9, Comparative Examples A6 to A11).

<Criterion for evaluation of the cleansing performance on each type of mascara>

| | |
|---|---|
| ◎ | The mascara is sufficiently removed. |
| ○ | The mascara is mostly removed. |
| ○Δ | The mascara is removed with a partial residue. |
| Δ | The mascara is not removed very much. |
| × | The mascara is not at all removed. |

(Evaluation method of feeling upon use)

An appropriate amount of the cleansing composition was applied evenly to the faces of 10 skilled panelists. They evaluated the feeling upon use (stickiness and sliminess) by sensory evaluation using five levels including "good", "slightly good", "not good nor bad", "not very good" and "not good". And then, judgment was made according to the following criterion.

[Criterion for evaluation]

◎ :  9 or 10 panelists evaluated "good" or "slightly good".

○:  7 or 8 panelists evaluated "good" or "slightly good".

∆:  4 to 6 panelists evaluated "good" or "slightly good".

✕:  3 or less panelists evaluated "good" or "slightly good".

(Example A1, comparative examples A1 to A5)

The cleansing compositions were prepared by the formulas shown in Table A-1. The cleansing performance on the film forming type aqueous mascara and the oily mascara, and also the feeling upon use were evaluated. The results are shown in Table A-1.

Table A-1

| | | Example | Comparative Example | | | | |
|---|---|---|---|---|---|---|---|
| | | A1 | A1 | A2 | A3 | A4 | A5 |
| (A) | Polyoxypropylene (9) diglyceryl ether : compound (a) | 10 | 0 | 30 | 10 | 5 | 10 |
| | Purified water | 75 | 85 | 55 | 90 | 25 | 5 |
| | 1,3-butylene glycol | 0 | 0 | 0 | 0 | 0 | 70 |
| (B) | Isododecane | 15 | 15 | 15 | 0 | 70 | 15 |
| | Total | 100 | 100 | 100 | 100 | 100 | 100 |
| | Concentration of compound (a) in (A) (% by weight) | 12 | 0 | 35 | 10 | 17 | 12 |
| | Weight ratio (A):(B) | 85:15 | 85:15 | 85:15 | 100:0 | 30:70 | 85:15 |
| | Evaluation | | | | | | |
| | Cleansing performance on the film forming type aqueous mascara | ◎ | ∆ | ∆ | ○∆ | ◎ | ∆ |
| | Cleansing performance on the oily mascara | ◎ | ◎ | ◎ | ✕ | ◎ | ◎ |
| | Feeling upon use | ◎ | ∆ | ○ | ◎ | ✕ | ∆ |

From the results shown in Table A-1, Example A1 containing the two components (A) and (B) at the ratio specified in the present invention was able to efficiently remove both the film forming type aqueous mascara with a large amount of the alkyl acrylate copolymer and the oily mascara, and showed an excellent feeling upon use. In contrast, the composition not containing compound (a) in component (A) (Comparative Example A1) or the composition containing more than 25 % by weight of compound (a) based on component (A) (Comparative Example A2) was able to remove the oily mascara, but the cleansing performance thereof on the film forming type mascara was poor. Moreover, the composition not containing the component (B) (Comparative Example A3) showed a certain level of the cleansing performance on the film forming type mascara but an insufficient cleansing performance on the oily mascara. It was revealed that not only the cleansing performance on the oilymascara but also the cleansing performance on the film forming type mascara with a large amount of the alkyl acrylate copolymer were remarkably improved by combining component (A) and component (B).

Although the composition containing both component (A) and component (B) but having the weight ratio (A):(B) outside the range of 97:3 to 40:60 (Comparative Example A4) was able to effectively remove both the oily mascara and the film forming type mascara, the feeling upon use thereof was not good due to a strong oily feeling. Moreover, the composition having less than 10% by weight of water (Comparative Example A5) was not able to remove the mascara containing a large amount of an alkyl acrylate copolymer, and the feeling upon use thereof was not good due to a slimy and sticky feeling.

(Examples A2 to A9, Comparative Examples A6 to A7)

The cleansing compositions further containing component (C) of the present invention were prepared by the formulas shown in Table A-2. The cleansing performance on the film forming type aqueous mascara and on the oily mascara, and also the feeling upon use were evaluated. The results are shown in Table A-2.

Table A-2 (1)

| | | | Example | | | | |
|---|---|---|---|---|---|---|---|
| | | | A2 | A3 | A4 | A5 | A6 |
| (A) | (a) | Polyoxypropylene (9) diglyceryl ether | 0 | 0 | 0 | 0 | 5 |
| | | Polyoxypropylene (10) glyceryl ether | 0 | 0 | 0 | 10 | 0 |
| | | Polyoxypropylene (10) methyl glucoside | 0 | 0 | 10 | 0 | 0 |
| | | Polypropylene glycol (10) | 10 | 10 | 0 | 0 | 0 |
| | | Glycerin | 0 | 0 | 0 | 0 | 0 |
| | | Ethanol | 0 | 0 | 0 | 0 | 0 |
| | | 1,3-butylene glycol | 5 | 5 | 5 | 5 | 5 |
| | | Aqueous solution of potassium hydroxide (48%) | 0.017 | 0.017 | 0.017 | 0.017 | 0.017 |
| | | Purified water | 64.363 | 64.363 | 64.363 | 64.363 | 74.318 |
| | | Phenoxy ethano | 0.3 | 0.3 | 0.3 | 0.3 | 0.3 |
| | | Paraoxy methyl benzoate | 0.2 | 0.2 | 0.2 | 0.2 | 0.2 |
| | (a') | Polyoxyethylene (3.5) polyoxypropylene (7) butyl ether | 0 | 0 | 0 | 0 | 0 |
| | | Polypropylene glycol (34) | 0 | 0 | 0 | 0 | 0 |
| (B) | | Isododecane | 0 | 15 | 20 | 20 | 15 |
| | | Isononyl isononate | 15 | 0 | 0 | 0 | 0 |
| | | Isopropyl palmitate | 5 | 5 | 0 | 0 | 0 |
| (C) | | PEMULEN TR-2 *1 | 0.04 | 0.04 | 0.04 | 0.04 | 0.04 |
| | | SPS *2 | 0.08 | 0.08 | 0.08 | 0.08 | 0.08 |
| Surfactant | | Polyethylene glycol (12) monolaurate | 0 | 0 | 0 | 0 | 0.045 |
| | | Polyoxyethylene (20) sorbitan monooleate | 0 | 0 | 0 | 0 | 0 |
| Total | | | 100 | 100 | 100 | 100 | 100 |
| | | Concentration of compound (a) in (A) (% by weight) | 13 | 13 | 13 | 13 | 6 |
| | | Weight ratio (A) : (B) | 79:20 | 79:20 | 79:20 | 79:20 | 84 : 15 |
| | | Evaluation | | | | | |
| | | Cleansing performance on the film forming type aqueous mascara | ○ | ◎ | ◎ | ◎ | ◎ |
| | | Cleansing performance on the oily mascara | ○Δ | ○ | ◎ | ◎ | ◎ |
| | | Feeling upon use | ○ | ○ | ○ | ◎ | ◎ |

Table A-2 (2)

| | | | Example | | | Comparative Example | |
|---|---|---|---|---|---|---|---|
| | | | A7 | A8 | A9 | A6 | A7 |
| (A) | (a) | Polyoxypropylene (9) diglyceryl ether | 5 | 5 | 5 | 0 | 0 |
| | | Polyoxypropylene (10) glyceryl ether | 0 | 0 | 0 | 0 | 0 |
| | | Polyoxypropylene (10) methyl glucoside | 0 | 0 | 0 | 0 | 0 |
| | | Polypropylene glycol (10) | 0 | 0 | 0 | 0 | 0 |
| | | Glycerin | 5 | 0 | 0 | 0 | 0 |
| | | Ethanol | 0 | 10 | 0 | 0 | 0 |
| | | 1,3-butylene glycol | 5 | 5 | 5 | 5 | 5 |
| | | Aqueous solution of potassium hydroxide (48%) | 0.017 | 0.017 | 0.017 | 0.017 | 0.017 |
| | | Purified water | 69.318 | 64.318 | 72.363 | 69.318 | 69.318 |
| | | Phenoxy ethanol | 0.3 | 0.3 | 0.3 | 0.3 | 0.3 |
| | | Paraoxy methyl benzoate | 0.2 | 0.2 3 | 0.2 | 0.2 | 0.2 |
| | (a') | Polyoxyethylene (3.5) polyoxypropylene (7) butyl ether | 0 | 0 | 0 | 10 | 0 |
| | | Polypropylene glycol (34) | 0 | 0 | 0 | 0 | 10 |
| (B) | | Isododecane | 15 | 15 | 15 | 15 | 15 |
| | | Isononyl isononate | 0 | 0 | 0 | 0 | 0 |
| | | Isopropyl palmitate | 0 | 0 | 0 | 0 | 0 |
| (C) | | PEMULEN TR-2 *1 | 0.04 | 0.04 | 0.04 | 0.04 | 0.04 |
| | | SPS *2 | 0.08 | 0.08 | 0.08 | 0.08 | 0.08 |
| Surfactant | | Polyethylene glycol (12) monolaurate | 0.045 | 0 | 2 | 0.045 | 0.045 |
| | | Polyoxyethylene (20) sorbitan monooleate | 0 | 0.045 | 0 | 0 | 0 |
| | | Total | 100 | 100 | 100 | 100 | 100 |
| | | Concentration of compound (a) in (A) (% by weight) | 6 | 6 | 6 | 0 | 0 |
| | | Weight ratio (A) : (B) | 84 : 15 | 84 : 15 | 84 : 15 | - | - |
| | | Evaluation | | | | | |
| | | Cleansing performance on the film forming type aqueous mascara | ◎ | ◎ | ○Δ | Δ | A |
| | | Cleansing performance on the oily mascara | ◎ | ◎ | ○Δ | Δ | ○ |
| | | Feeling upon use | ◎ | ◎ | ◎ | ○ | ○ |

The origins of the materials used are shown below.

*1: acrylic acid·alkyl methacrylate copolymer: trade name PEMULEN TR-2, Noveon, Inc.

*2: hydroxyl ethyl cellulose hydroxy propyl stearyl ether hydroxyl propyl sodium sulfonate (JP-A No. 9(1997)-235301).

Examples A2 to A9 were the liquid cleansing compositions wherein an aqueous thickening agent having a hydrophobic part as component (C) was added. According to these Examples, components (A) and (B) became a homogeneous emulsion, therefore, the cleansing performance was able to be evaluated by direct application without use of a cotton sheet. From the results shown in Table A-2, it was revealed that Examples A2 to A9 containing the two components (A) and (B) at the ratio specified in the present invention were able to remove effectively both the film forming type mascara having a large amount of an alkyl acrylate copolymer and the oilymascara, and to obtain an excellent feeling upon use.

Example A5 containing polyoxypropylene (10) glycerylether as compound (a) , and Examples A6 to A9 containing polyoxypropylene (9) diglyceryl ether as compound (a) showed a better feeling upon use than Examples A2 to A4 which contained other compounds as compound (a) . Moreover, Examples A5 to A9 showed particularly an excellent feeling upon use and a comfortable moist feeling.

In contrast, Comparative Example A6 containing a compound further having an alkyl group having 4 carbon atoms and a polyoxyethylene structure together with the polyoxypropylene structure showed poorer cleansing performances on both the film forming type mascara and the oily mascara than the Examples. Moreover, Comparative Example A7 containing polypropylene glycol (34) which is not water-soluble due to the large addition number of propylene oxide and does not correspond to compound (a) of the present invention was able to remove the oily mascara, but showed a poorer cleansing performance on the film forming type mascara than the Examples.

Moreover, Example A7 containing glycerin showed an excellent feeling with a more moist feeling than Example A6. Example A8 containing ethanol showed an excellent feeling upon use with a more fresh feeling than Example A6.

Example A3 containing more than 50% by weight of isododecane in oil phase (B) showed excellent cleansing performances on both the film forming type mascara and the oily mascara compared with Example A2 containing no isododecane. Furthermore, in Example A4 containing more than 80% by weight of isododecane in oil phase (B), the cleansing performance on the oily mascara was more excellent than Example A3.

Moreover, Examples A6 to A8 containing 0.045% by weight of the surfactant and Example A9 containing 2% by weight thereof showed cleansing performances on both the film forming type mascara and the oily mascara, and good comfort to the skin. Particularly Examples A6 to A8 containing 0.045% by weight of the surfactant showed better cleansing performances on both the film forming type mascara and the oily mascara than Example A9 containing 2% by weight of the surfactant.

(Comparative Example A8)

The cleansing composition was prepared by the formula shown in Table A-3, and the cleansing performances on the mascara were evaluated. The results are shown in Table A-3.

Table A-3

|  |  | Comparative Example A8 |
|---|---|---|
| Composition | Hexaglyceryl monomyristate | 4 |
|  | Polyoxypropylene (15) methyl glucoside | 15 |
|  | Sodium polyacrylate | 0.5 |
|  | Purified water | 80.5 |
|  | Total | 100 |
| Evaluation | Cleansing performance on the film forming type aqueous mascara | ○Δ |
|  | Cleansing performance on the oily mascara | ✕ |

It was revealed that the composition containing a polyoxypropylene methyl glucoside and a nonionic surfactant having HLB value of more than 10 (hexaglyceryl monomyristate), which cprresponds to the example of JP-A No. 9(1997)-143028, showed a cleansing performance on the film forming type mascara to some extent but showed a poorer cleansing performance on the oily mascara.

(Comparative Example A9)

The cleansing composition was prepared by the formula shown in Table A-4, and the cleansing performances on the mascara were evaluated. The results are shown in Table A-4.

Table A-4

| | | Comparative Example A9 |
|---|---|---|
| Composition | Polypropylene glycol (10) | 5 |
| | Ethanol | 15 |
| | Glycerin | 5 |
| | Polyoxyethylene (20) sorbitan monooleate | 3 |
| | Purified water | 72 |
| | Total | 100 |
| Evaluation | Cleansing performance on the film forming type aqueous mascara | ○Δ |
| | Cleansing performance on the oily mascara | × |

It was revealed that the composition containing polyalkylene glycol, ethanol, a water-soluble polyhydric alcohol, a surfactant, and water, which corresponds to the example of JP-A No. 2 (1990)-104511, showed a cleansing performance on the film forming type mascara to some extent but showed a poorer cleansing performance on the oily mascara.

(Comparative Example A10)

The cleansing composition was prepared by the formula shown in Table A-5, and the cleansing performances on the mascara were evaluated. The results are shown in Table A-5.

Table A-5

| | | Comparative Example A10 |
|---|---|---|
| Composition | Isododecane | 15 |
| | Alkyl (C8 to C16) glucoside | 3 |
| | Glycerin | 5 |
| | PEMULEN TR-2 *1 | 0.04 |
| | Aqueous solution of potassium hydroxide (48%) | 0.017 |
| | Phenoxy ethanol | 0.3 |
| | Paraoxy methyl benzoate | 0.2 |
| | Purified water | 76.443 |
| | Total | 100 |
| Evaluation | Cleansing performance on the film forming type aqueous mascara | Δ |
| | Cleansing performance on the oily mascara | ◎ |

The composition containing an emulsion including an acrylic acid-alkyl methacrylate copolymer (Noveon, Inc., trade name: PEMULEN TR-2), an oil and water, and a non-emulsifying surfactant (alkyl (C8 to C16) glucoside) , which corresponds to the example of JP-A No. 11 (1999)-503461. It was similar to the case of comparative example A1, and it was able to remove the oily mascara but showed a poorer cleansing performance on the film forming type mascara containing a large amount of an alkyl acrylate copolymer than the Examples because it did not contain compound (a) used in the present invention.

(Comparative Example A11)

The cleansing composition was prepared by the formula shown in Table A-6, and the cleansing performances on the mascara were evaluated. The results are shown in Table A-6.

Table A-6

|  |  | Comparative Example A11 |
|---|---|---|
| Composition | Light isoparaffin | 20 |
|  | Liquid paraffin | 50 |
|  | Polyoxyethylene (15) cetyl ether | 1.5 |
|  | Polyoxyethylene (5) cetyl ether | 1 |
|  | Polyoxyethylene (2) behenyl ether | 1 |
|  | Polyoxyethylene (20) sorbitan tristearate | 1 |
|  | Paraoxy methyl benzoate | 0.3 |
|  | Propylene glycol | 3 |
|  | Purified water | 22.2 |
|  | Total | 100 |
| Evaluation | Cleansing performance on the film forming type aqueous mascara | Δ |
|  | Cleansing performance on the oily mascara | ○ |

The composition having an isoparaffin emulsified in water with a polyoxyethylene alkyl ether-based surfactant, which corresponds to the example of JP-A No. 5-163116, was able to remove the oily mascara, but showed a poorer cleansing performance on the film forming type aqueous mascara containing a large amount of an alkyl acrylate copolymer than the Examples.

Example B series: cleansing sheet

(Cleansing performance evaluation method)

For the evaluation of the cleansing performance, a film forming type aqueous mascara containing an alkyl acrylate copolymer, a film forming type oily mascara, and oily mascara were used.

A predetermined amount of the mascara was applied on a glass slide evenly to form a circle of 1.2 cm in diameter and dried by allowing to stand for 12 hours, thus obtaining the test stains. 0. 0070 g of TESTIMO MASCARA EFFECT CURLLONG BK18 (trade name) produced by Kanebo, Ltd. as the film forming type aqueous mascara, 0.0045 g of (trade name) AUBE REAL EFFECT MASCARA BK941 produced by Kao Corporation as the film forming type oily mascara, and 0.0045 g of SPORTS BEAUTY FASIO POWER STAY MASCARA CURLLONG BK001 (tradename) produced by KOS-É Corporation as the oily mascara were applied. Because of the difference in the liquid property of each mascara, the amount of coating of each mascara is different.

The cleansing sheet was placed on the test stains and lightly pressed thereon for 5 seconds. Next, test stains were wiped off with a constant pressure (100 g/cm$^2$) to count the number of wiping actions necessary to remove the mascara.

<Criterion for evaluation of the cleansing performance on the film forming type aqueous mascara>

| ◎ | 14 times or less |
|---|---|
| ○ | 15 to 19 times |
| ○Δ | 20 to 24 times |
| Δ | 25 to 29 times |
| × | 30 times or more |

<Criterion for evaluation of the cleansing performance on the film forming type oily mascara and the oily mascara>

| ◎ | 5 times or less |
|---|---|
| ○ | 6 to 10 times |

(continued)

| ○Δ | 11 to 15 times |
| Δ | 16 to 20 times |
| × | 21 times or more |

(Evaluation method of the texture of the skin after use)

10 skilled panelists wiped the face with the cleansing sheets, and then evaluated textures to the skin after use by sensory evaluation.

[Criterion for evaluation]

◎ : 9 or 10 panelists evaluated that the oily feeling was absent without stickiness.
○: 7 or 8 panelists evaluated that the oily feeling was absent without stickiness.
Δ: 5 or 6 panelists evaluated that the oily feeling was absent without stickiness.
×: 4 or less panelists evaluated that the oily feeling was absent without stickiness.

(Evaluation method of odor)

10 skilled panelists wiped the face with the cleansing sheets, and then evaluated odors at the time of or after wiping by sensory evaluation.

[Criterion for evaluation]

○: 8 or more panelists evaluated that it is an odor acceptable for use.
Δ: 5 to 7 panelists evaluated that it is an odor acceptable for use.
x: 4 or less panelists evaluated that it is an odor acceptable for use.

(Viscosity)

The viscosity of the prepared emulsion composition was measured by a BM type viscometer (produced by TOKIMEC Inc.) under the conditions of 6 rpm and 30°C using Rotor No. 2.

(Evaluation of emulsion composition stability)

The prepared emulsion composition was put into a glass bottle having a screw cap, and stored in a temperature-controlled room of 30°C. The change of external appearance was observed after 1 day, 3 days, 7 days, 10 days, 14 days and 1 month.

(Evaluation method of texture to the skin at the time of wiping off)

The 10 skilled panelists evaluated textures to the skin at the time of wiping the face with each of the cleansing sheets by sensory evaluation.

[Criterion for evaluation]

◎ : 9 or 10 panelists evaluated that the cleansing sheet showed good contact to the skin without slippage.
○: 7 or 8 panelists evaluated that the cleansing sheet showed good contact to the skin without slippage.
Δ: 5 or 6 panelists evaluated that the cleansing sheet showed good contact to the skin without slippage.
×: 4 or less panelists evaluated that the cleansing sheet showed good contact to the skin without slippage.

(Evaluation method of quality stability at the time of stacking the sheets: the ratio of impregnation immediately after the preparation and after the storage)

Nonwoven fabric sheets cut in a 75 mm × 200 mm size were folded into the form of Z, and 40 sheets impregnated with the emulsion composition were stacked. The ratio of impregnation immediately after preparation was then measured. Next, the stacked sheets were put into an aluminum pillow, sealed, and stored at 50°C for 1 month. Thereafter, after returning them to room temperature, the ratio of impregnation and the cleansing performance of the second sheet from the top of the stack and the second sheet from the bottom of the stack were evaluated. The ratio of impregnation can be calculated by the following equation after measuring the weight of the impregnated sheets, washing the sheets with a detergent, rinsing with water, drying, and measuring the weight of the dried sheets.

```
Ratio of impregnation % = (impregnated sheet weight – dried sheet

weight) × 100/dried sheet weight
```

[Criterion for evaluation]

◎ : The change of the ratio of impregnation after storage is within ± 25% with respect to the ratio of impregnation immediately after preparation.
○: The change of the ratio of impregnation after storage is more than ± 25% and ±40% or less with respect to the ratio of impregnation immediately after preparation.
Δ: The change of the ratio of impregnation after storage is more than ± 40% and ±60% or less with respect to the ratio of impregnation immediately after preparation.
×: The change of the ratio of impregnation after storage is more than ± 60% with respect to the ratio of impregnation immediately after preparation.

(Examples B1 to B7, Comparative Examples B1 to B5)

The emulsion compositions were prepared by the formulas shown in Table B-1. Then, test samples were prepared by impregnating a nonwoven fabric made of 100% cotton, having a 0.15 g/cm$^3$ density and a 60 g/m$^2$ average basis weight with 3.5 g of the emulsion composition with respect to 1 g of the nonwoven fabric. The results are shown also in Table B-1. The amount of each component is expressed in "parts by weight" when the total amount is 100.

(Emulsion composition production method)

An aqueous thickening agent of component (C) was added to purified water so as to be dispersed homogeneously by agitating with a Disper at 1,200 rpm for 30 minutes. While agitating with a propeller at 200 rpm, aqueous solution of potassium hydroxide as the neutralizing agent was added and stirred for 10 minutes for neutralization and thickening. While the agitation continued, other component (A), component (a') and the surfactant were added and dissolved. While the agitation further continued, the oil of component (B) was dropped slowly and stirred for 2 hours. Thereafter, an emulsion composition was obtained by agitating with Agi homo mixer (TOKUSHU KIKA KOGYO CO., LTD.) at 4,500 rpm for 30 minutes.

Table B-1 (1)

| | | | Example | | | | | | |
|---|---|---|---|---|---|---|---|---|---|
| | | | B1 | B2 | B3 | B4 | B5 | B6 | B7 |
| (A) | a | Polyoxypropylene (9) diglyceryl ether | 10 | 10 | | 10 | | 10 | |
| | | Polypropylene glycol (10) | | | 10 | | 10 | | 10 |
| | | 1,3-butylene glycol | | | | | 5 | | |
| | | Ethanol | | | | | | | |

(continued)

| | | | Example | | | | | | |
|---|---|---|---|---|---|---|---|---|---|
| | | | B1 | B2 | B3 | B4 | B5 | B6 | B7 |
| | | Aqueous solution of potassium hydroxide (48%) | 0.023 | 0.023 | 0.023 | 0.023 | 0.023 | 0.038 | 0.023 |
| | | Purified water | Balance (the amount to make the total amount be 100) | | | | | | |
| | a' | Polyethylene glycol (20) polypropylene glycol (10) | | | | | | | |
| (B) | b | IP solvent 1620 *3 | 15 | | | | | | |
| | | IP solvent 2028 *4 | | | 5 | 5 | 5 | | 5 |
| | | MARCASOL R *5 | | 15 | | | | 15 | |
| | | Decamethyl cyclopentasiloxane (boiling point 205°C) | | | 5 | 5 | 5 | | 5 |
| | | Isopropyl palmitate (boiling point 332°C) | | | 3 | 3 | 3 | | 3 |
| | | Liquid isoparaffin | | | | | | | |
| (C) | | PEMULEN TR-2 *1 | 0.05 | 0.05 | 0.05 | 0.05 | 0.05 | 0.05 | 0.05 |
| | | CARBOPOL ETD *6 | | | | | | 0.025 | |
| | | SPS *2 | 0.1 | 0.1 | 0.1 | 0.1 | 0.1 | 0.12 | 0.1 |
| Surfac tant | | POE (10) laurate | | | | | | | 0.045 |
| | | POE (15) cetyl ether | | | | | | | |
| | | POE (6) cetyl ether | | | | | | | |
| | | POE (20) sorbitan monooleate | | | | | | | |
| | | Sorbitan monooleate | | | | | | | |
| | | Evaluation | | | | | | | |
| | | Cleansing performance on the film forming type aqueous mascara | ◎ | ◎ | ○ | ○ | ○ | ○ | ○ |
| | | Cleansing performance on the film forming type oily mascara | ◎ | ◎ | ○ | ○ | ○ | ○ | ○ |
| | | Cleansing performance on the oily mascara | ◎ | ◎ | ○ | ○ | ○ | ○ | ○ |
| | | Texture to the skin after use | ○ | ◎ | ○ | ◎ | ○ | ○ | ○ |
| | | Odor | Δ | ○ | ○ | ○ | ○ | ○ | ○ |
| | | Viscosity (mPa·s) | 650 | 625 | 600 | 625 | 475 | 2000 | 600 |
| | | State of the emulsion composition after one month | *S | *S | *S | *S | *S | *N | *S |

*S: separated    *N: non-separated

Table B-1 (2)

| | | | Comparative Example | | | | |
|---|---|---|---|---|---|---|---|
| | | | B1 | B2 | B3 | B4 | B5 |
| (A) | a | Polyoxypropylene (9) diglyceryl ether | | | | | |
| | | Polypropylene glycol (10) | | | | 10 | 10 |
| | | 1,3-butylene glycol | | 5 | | | |
| | | Ethanol | | 15 | | | |
| | | Aqueous solution of potassium hydroxide (48%) | | | 0.023 | 0.023 | |
| | | Purified water | Balance (the amount to make the total amount be 100) | | | | |
| | a' | Polyethylene glycol (20) polypropylene glycol (10) | | | 5 | | |
| (B) | b | IP solvent 1620 *3 | 25 | | | | |
| | | IP solvent 2028 *4 | | | 5 | | 5 |
| | | MARCASOL R *5 | | | | | |
| | | Decamethyl cyclopentasiloxane (boiling point 205°C) | | | 5 | 5 | 5 |
| | | Isopropyl palmitate (boiling point 332°C) | | | 3 | 5 | 3 |
| | | Liquid isoparaffin | | | | 5 | |
| (C) | | PEMULEN TR-2 *1 | | | 0.05 | 0.05 | |
| | | CARBOPOL ETD *6 | | | | | |
| | | SPS *2 | | | 0.1 | 0.1 | |
| Surfa ctant | | POE (10) laurate | | | | | |
| | | POE (15) cetyl ether | 1.5 | | | | |
| | | POE (6) cetyl ether | 1 | | | | |
| | | POE (20) sorbitan monooleate | | 3 | | | 1.2 |
| | | Sorbitan monooleate | | | | | 1.2 |
| | | Evaluation | | | | | |
| | | Cleansing performance on the film forming type aqueous mascara | Δ | Δ | × | ○Δ | ○Δ |
| | | Cleansing performance on the film forming type oily mascara | ○Δ | × | ○Δ | ○Δ | Δ |
| | | Cleansing performance on the oily mascara | ◎ | × | ○ | ○Δ | Δ |
| | | Texture to the skin after the use | Δ | Δ | ○ | Δ | Δ |
| | | Odor | Δ | ○ | ○ | ○ | ○ |
| | | Viscosity (mPa·s) | 300 | 12 | 600 | 800 | 1200 |
| | | State of the emulsion composition after one month | *S | *7 | *S | *S | *N |

*S: separated      *N: non-separated
*7: Since it did not contain an oil, it was a homogeneous solution.

The origins of the used materials are shown below. *1: acrylic acid-alkyl methacrylate copolymer: trade name PEMULEN TR-2, Noveon, Inc.

*2: hydroxyl ethyl cellulose hydroxy propyl stearyl ether hydroxyl propyl sodium sulfonate (JP-A No. 9(1997)-235301).
*3: light liquid isoparaffin (boiling point 166 to 202°C) : trade name IP solvent 1620, Idemitsu Petrochemical Co., Ltd.
*4: light liquid isoparaffin (boiling point 213 to 262°C) : trade name IP solvent 2028, Idemitsu Petrochemical Co., Ltd.
*5: light fluid isoparaffin (boiling point 175 to 185°C) : trade name MARCASOL R, Maruzen Petrochemical Co., Ltd.
*6: Acrylic acid-alkyl methacrylate copolymer: trade name CARBOPOL ETD, Noveon, Inc.

From the results shown in Table B-1, Examples B1 to B7 which were the nonwoven fabrics impregnated with the example oil-in-water emulsion composition of the present invention were able to remove the hard-to-remove film forming type aqueous mascara, the film forming type oily mascara, and the oily mascara conveniently, quickly, and effectively, and showed good texture to the skin after use. As to the odor, the examples using the IP solvent 1620 including an isoparaffin having 8 and 9 carbon atoms showed a slightly poor evaluation.

In Example B6 using a relatively stable emulsion composition having a relatively high viscosity, which did not separate after 1 month of storage, the intended benefits of the present invention was obtained. However, compared with Example B2 using the same amount of the same compound (a) and oil (b) as Example B6, Example B2 using the emulsion composition which separated after 1 month of storage showed more excellent effects than Example B6.

Moreover, Example B5 containing a 1,3-butylene glycol, showed a more moist feeling upon use than Example B3 using the same amount of the same compound (a) and oil (b) as Example B5. Example B7 containing 0.045% by weight of the surfactant showed better comfort to the skin than Example B3 using the same amount of the same compound (a) and oil (b) as Example B7.

In contrast, Comparative Example B1 was prepared by using surfactants which are commonly used as an emulsifier instead of the aqueous thickening agent and not using compound (a) . It corresponds to the example of JP-A No. 5(1993)-163116, showed a poor cleansing performance on the mascara containing a film forming component and a bad texture to the skin after use. Moreover, Comparative Example B2 was prepared by using a surfactant which is commonly used as an emulsifier instead of the aqueous thickening agent, using component (a') , and not using component (B) to provide the oil phase. It corresponds to JP-A No. 2(1990)-104511, and was able to remove neither the oily mascara nor the film forming type aqueous mascara, and showed a bad texture to the skin after use.

Comparative Example B3 not including compound (a) showed a poor cleansing performance on mascara containing the film forming component. Moreover, Comparative Example B4 containing less than 50% by weight of the oil (b) having a boiling point of 160 to 300°C in the oil phase showed a poorer cleansing performance on the mascara and a poorer texture to the skin than the Examples. In Comparative Example B5 emulsified using the conventional emulsifier instead of component (C), the oil was hard to release due to the stable emulsion so that the removing performance, in particular, the removing performance on the oily mascara was poor.

(Examples B8 to B11, Comparative Examples B6 to B8)

The samples were prepared by impregnating the nonwoven fabric shown in Table B-2 with the emulsion compositions. For Examples B8 to B11 and Comparative Examples B6 and B7, the emulsion composition prepared in Example B2 was used. The emulsion composition prepared in Example B6 was used for Comparative Example B8. Evaluation results of the texture to the skin at the time of wiping and the stability of quality at the time of stacking the sheets are also shown in Table B-2. Example B2 is also shown in Table B-2.

Table B-2

| | | Example | | | | | Comparative Example | | |
|---|---|---|---|---|---|---|---|---|---|
| | | B2 | B8 | B9 | B10 | B11 | B6 | B7 | B8 |
| Non woven fabric composition (%) | Cotton | 100 | 100 | 100 | | | 100 | 50 | 100 |
| | Rayon | | | | 100 | 80 | | | |
| | PET | | | | | 20 | | 50 | |
| Density of the nonwoven fabric (g/cm$^3$) | | 0.15 | 0.19 | 0.25 | 0.19 | 0.20 | 0.12 | 0.15 | 0.12 |
| Basis weight of the nonwoven fabric (g/m$^2$) | | 60 | 60 | 100 | 60 | 80 | 30 | 60 | 30 |
| Amount of impregnating Liquid (g/nonwoven fabric g) | | 3.5 | 3.5 | 3.5 | 3.5 | 3.5 | 3.2 | 3.5 | 3.5 |

(continued)

| | | Example | | | | | Comparative Example | | |
| --- | --- | --- | --- | --- | --- | --- | --- | --- | --- |
| | | B2 | B8 | B9 | B10 | B11 | B6 | B7 | B8 |
| Texture to the skin at the time of wiping off | | ◎ | ◎ | ○ | ○ | ○ | ○ | △ | ○ |
| Ratio of impregnation after storage (The second sheet from the top of the stack) | | ○ | ◎ | ◎ | ◎ | ○ | △ | △ | △ |
| Ratio of impregnation after storage (The second sheet from the bottom of the stack) | | ○ | ◎ | ◎ | ◎ | ○ | △ | △ | △ |
| Cleansing performance after storage (The second sheet from the top of the stack) | Film forming type aqueous mascara | ◎ | ◎ | ◎ | ◎ | ◎ | ○ | ○ | △ |
| | Film forming type oily mascara | ◎ | ◎ | ◎ | ◎ | ◎ | △ | △ | △ |
| Cleansing performance after storage (The second sheet from the bottom of the stack) | Film forming type aqueous mascara | ◎ | ◎ | ◎ | ◎ | ◎ | ◎ | ◎ | ○ |
| | Film forming type oily mascara | ◎ | ◎ | ◎ | ◎ | ◎ | ◎ | ◎ | ○ |

The cleansing sheets of both the Examples and the Comparative Examples showed excellent cleansing performances on the mascara containing the film forming component immediately after preparation. From the results shown in Table B-2, Examples B2, B8 to B11 using the nonwoven fabric which was made of a fiber having a cellulose content of at least about 70% by weight and had a density of about 0.15 to 0.3 g/cm$^3$ showed excellent cleansing performances after storage with the sheets stacked as well as immediately after preparation, and thus has excellent stability of quality by stacking. Moreover, the cleansing sheets of the Examples showed excellent texture to the skin at the time of wiping.

In contrast, according to Comparative Example B6 using the nonwoven fabric having a relatively low density, the composition of the impregnating liquid varied after storage, so that the cleansing performance after storage varied between the second sheet from the top of the stack and the second sheet from the bottom of the stack. Moreover, according to Comparative Example 7 using the nonwoven fabric containing 50% of PET (polyethylene terephthalate) as the hydrophobic fiber, the ratio of impregnation varied after storage, so that the cleansing performance after storage varied between the second sheet from the top of the stack and the second sheet from the bottom of the stack. Comparative Example B7 also showed a poor texture to the skin at the time of wiping.

Furthermore, Comparative Example B8 was prepared by using a stable oil-in-water emulsion composition and a nonwoven fabric having a relatively low density. It was revealed that the ratio of impregnation may be varied depending on the sheet to be impregnated. Even in the case of using a stable oil-in-water emulsion composition, a change of the cleansing performance and loss of the stability of quality may occur when the sheets are stacked and stored.

**Claims**

1.    A cleansing composition comprising the following components (A) and (B):

   (A) an aqueous phase containing 1 to 25% by weight, in the aqueous phase, of a water-soluble compound (a) which has a polyoxypropylene structure having 3 or more addition number of propylene oxide but neither an alkyl group having 4 or more carbon atoms nor a polyoxyethylene structure; and
   (B) an oil phase which is liquid at 30°C and contains 50% by weight or more of an oil (b) having a boiling point of 160 to 300°C at 1,013.25 hPa, selected from light liquid isoparaffin and volatile silicone, and

   further comprising more than 10% by weight of water based on the total composition,
   wherein the weight ratio (A):(B) of the aqueous phase (A) and the oil phase (B) is 97:3 to 40:60,.

2. The cleansing composition according to claim 1, further comprising 0.01 to 5% by weight of (C) an aqueous thickening agent.

3. The cleansing composition according to claim 2, wherein component (C) is an aqueous thickening agent having a hydrophobic part.

4. The cleansing composition according to any of claims 1 to 3, wherein the water-soluble compound (a) in component (A) is a propylene oxide addition product of glycerin and/or polyglycerin.

5. The cleansing composition according to any of claims 1 to 4, wherein component (B) has a viscosity of 10 mPa·s or less at 30°C.

6. The cleansing composition according to claim 1, further comprising a surfactant in an amount of 2% by weight or less based on the total composition.

7. A cleansing sheet comprising a nonwoven fabric which comprises a fiber having a cellulose content of at least 70% by weight and has a density of 0.15 to 0.3 g/cm$^3$ impregnated with an oil-in-water emulsion composition comprising the following components (A), (B), and (C):

   (A) an aqueous phase containing 1 to 25% by weight, in the aqueous phase, of a water-soluble compound (a) which has a polyoxypropylene structure having 3 or more addition number of propylene oxide but neither an alkyl group having 4 or more carbon atoms nor a polyoxyethylene structure;
   (B) an oil phase containing 50% by weight or more of an oil (b) having a boiling point of 160 to 300°C at 1,013.25 hPa selected from light liquid isoparaffin and volatile silicone; and
   (C) 0.01 to 1% by weight of an aqueous thickening agent having a hydrophobic part,

   wherein the weight ratio (A):(B) of the aqueous phase (A) and the oil phase (B) is 97:3 to 40:60.

8. The cleansing sheet according to claim 7, wherein the composition further comprises a surfactant in an amount of 2% by weight or less based on the total composition.

9. A method of removing makeup from an area on the skin applied with makeup cosmetics by using a cleansing composition comprising the following components (A) and (B) :

   (A) an aqueous phase containing 1 to 25% by weight, in the aqueous phase, of a water-soluble compound (a) which has a polyoxypropylene structure having 3 or more addition number of propylene oxide but neither an alkyl group having 4 or more carbon atoms nor a polyoxyethylene structure; and
   (B) an oil phase which is liquid at 30°C, and contains 50% by weight or more of an oil (b) having a boiling point of 160 to 300°C at 1,013.25 hPa selected from light liquid isoparaffin and volatile silicone; and

   further comprising more than 10% by weight of water based on the total composition,
   wherein the weight ratio (A):(B) of the aqueous phase (A) and the oil phase (B) is 97:3 to 40:60.

10. The method of claim 9, wherein the composition further comprises a surfactant in an amount of 2% by weight or less based on the total composition.


**Patentansprüche**

1. Reinigungszusammensetzung, enthaltend die folgenden Komponenten (A) und (B):

   (A) eine wässrige Phase, enthaltend 1 bis 25 Gew.-% einer wasserlöslichen Verbindung (a), die eine Polyoxypropylenstruktur mit 3 oder mehr Additionszahlen von Propylenoxid, aber weder eine Alkylgruppe mit 4 oder mehr Kohlenstoffatomen noch eine Polyoxyethylenstruktur aufweist, in der wässrigen Phase und
   (B) eine Ölphase, die bei 30°C flüssig ist und 50 Gew.-% oder mehr eines Öls (b) mit einem Siedepunkt von 160 bis 300°C bei 1013,25 hPa enthält, ausgewählt aus leichtem flüssigen Isoparaffin und flüchtigem Silicon, und

   weiterhin enthaltend mehr als 10 Gew.-% Wasser, bezogen auf die Gesamtzusammensetzung,

worin das Gewichtsverhältnis (A):(B) der wässrigen Phase (A) und der Ölphase (B) 97:3 bis 40:60 ist.

2. Reinigungszusammensetzung nach Anspruch 1, weiterhin enthaltend 0,01 bis 5 Gew.-% eines wässrigen Verdickungsmittels (C).

3. Reinigungszusammensetzung nach Anspruch 2, worin die Komponente (C) ein wässriges Verdickungsmittel mit einem hydrophoben Teil ist.

4. Reinigungszusammensetzung nach einem der Ansprüche 1 bis 3, worin die wasserlösliche Verbindung (a) in der Komponente (A) ein Propylenoxid-Additionsprodukt von Glycerin und/oder Polyglycerin ist.

5. Reinigungszusammensetzung nach einem der Ansprüche 1 bis 4, worin die Komponente (B) eine Viskosität von 10 mPa·s oder weniger bei 30°C hat.

6. Reinigungszusammensetzung nach Anspruch 1, weiterhin umfassend ein Tensid in einer Menge von 2 Gew.-% oder weniger, bezogen auf die Gesamtzusammensetzung.

7. Reinigungslage, enthaltend ein Vlies, das eine Faser mit einem Cellulosegehalt von wenigstens 70 Gew.-% enthält und eine Dichte von 0,15 bis 0,3 g/cm$^3$ hat, imprägniert mit einer Öl-in-Wasser-Emulsionszusammensetzung, enthaltend die folgenden Komponenten (A), (B) und (C):

(A) eine wässrige Phase, enthaltend 1 bis 25 Gew.-% einer wasserlöslichen Verbindung (a), die eine Polyoxypropylenstruktur mit 3 oder mehr Additionszahlen von Propylenoxid, aber weder eine Alkylgruppe mit 4 oder mehr Kohlenstoffatomen noch eine Polyoxyethylenstruktur aufweist, in der wässrigen Phase und
(B) eine Ölphase, die 50 Gew.-% oder mehr eines Öls (b) mit einem Siedepunkt von 160 bis 300°C bei 1013,25 hPa enthält, ausgewählt aus leichtem flüssigen Isoparaffin und flüchtigem Silicon, und
(C) 0,01 bis 1 Gew.-% eines wässrigen Verdickungsmittels mit einem hydrophoben Teil,

worin das Gewichtsverhältnis (A):(B) der wässrigen Phase (A) und der Ölphase (B) 97:3 bis 40:60 ist.

8. Reinigungslage nach Anspruch 7, worin die Zusammensetzung weiterhin ein Tensid in einer Menge von 2 Gew.-% oder weniger enthält, bezogen auf die Gesamtzusammensetzung.

9. Verfahren zur Entfernung von Make-up von einer Fläche der Haut, die mit Make-up-Kosmetik versehen ist, durch Verwendung einer Reinigungszusammensetzung, umfassend die folgenden Komponenten (A) und (B):

(A) eine wässrige Phase, enthaltend 1 bis 25 Gew.-% einer wasserlöslichen Verbindung (a), die eine Polyoxypropylenstruktur mit 3 oder mehr Additionszahlen von Propylenoxid, aber weder eine Alkylgruppe mit 4 oder mehr Kohlenstoffatomen noch eine Polyoxyethylenstruktur aufweist, in der wässrigen Phase und
(B) eine Ölphase, die bei 30°C flüssig ist und 50 Gew.-% oder mehr eines Öls (b) mit einem Siedepunkt von 160 bis 300°C bei 1013,25 hPa enthält, ausgewählt aus leichtem flüssigen Isoparaffin und flüchtigem Silicon, und

weiterhin enthaltend mehr als 10 Gew.-% Wasser, bezogen auf die Gesamtzusammensetzung,
worin das Gewichtsverhältnis (A):(B) der wässrigen Phase (A) und der Ölphase (B) 97:3 bis 40:60 ist.

10. Verfahren nach Anspruch 9, worin die Zusammensetzung weiterhin ein Tensid in einer Menge von 2 Gew.-% oder weniger enthält, bezogen auf die Gesamtzusammensetzung.

**Revendications**

1. Composition de nettoyage comprenant les composants (A) et (B) suivants :

(A) une phase aqueuse contenant de 1 à 25 % en poids, dans la phase aqueuse, d'un composé hydrosoluble (a) présentant une structure de polyoxypropylène possédant 3 ou plus d'un nombre additionnel d'oxyde de propylène, mais ni un groupe alkyle ayant 4 ou plus d'atomes de carbone, ni une structure de polyoxyéthylène et
(B) une phase huileuse liquide à 30 °C et qui contient 50 % en poids ou plus d'une huile (b) présentant un point d'ébullition de 160 °C à 300 °C à 1013,25 hPa, choisie parmi l'isoparaffine liquide légère et la silicone volatile et

comprenant en outre plus de 10 % en poids d'eau par rapport à la totalité de la composition,

dans laquelle le rapport pondéral (A)/(B) de la phase aqueuse (A) à la phase huileuse (B) est de 97/3 à 40/60.

2. Composition de nettoyage selon la revendication 1, comprenant en outre de 0,01 % à 5 % en poids d'un agent épaississant aqueux (C).

3. Composition de nettoyage selon la revendication 2, dans laquelle le composant (C) est un agent épaississant aqueux ayant une partie hydrophobe.

4. Composition de nettoyage selon l'une quelconque des revendications 1 à 3, dans laquelle le composé hydrosoluble (a) du composant (A) est un produit d'addition d'oxyde de propylène de glycérine et/ou de polyglycérine.

5. Composition de nettoyage selon l'une quelconque des revendications 1 à 4, dans laquelle le composant (B) présente une viscosité de 10 mPa.s ou moins à 30 °C.

6. Composition de nettoyage selon la revendication 1, comprenant en outre un tensioactif en quantité de 2 % en poids ou moins par rapport à la composition totale.

7. Feuille de nettoyage comprenant un tissu non tissé qui se compose d'une fibre ayant un contenu cellulosique d'au moins 70 % en poids et présente une masse volumique de 0,15 à 0,3 g/cm$^3$ imprégnée d'une émulsion huile-dans-l'eau comprenant les composants (A), (B) et (C) suivants :

(A) une phase aqueuse contenant de 1 à 25 % en poids, dans la phase aqueuse, d'un composé hydrosoluble (a) présentant une structure de polyoxypropylène possédant 3 ou plus d'un nombre additionnel d'oxyde de propylène, mais ni un groupe alkyle ayant 4 ou plus d'atomes de carbone, ni une structure de polyoxyéthylène et
(B) une phase huileuse qui contient 50 % en poids ou plus d'une huile (b) présentant un point d'ébullition de 160 °C à 300 °C à 1013,25 hPa, choisie parmi l'isoparaffine liquide légère et la silicone volatile ; et
(C) de 0,01 % à 1 % en poids d'un agent épaississant aqueux présentant une partie hydrophobe,

dans laquelle le rapport pondéral (A)/(B) de la phase aqueuse (A) à la phase huileuse (B) est de 97/3 à 40/60.

8. Feuille de nettoyage selon la revendication 7, dans laquelle la composition comprend en outre un tensioactif en quantité de 2 % en poids ou moins par rapport à la totalité de la composition.

9. Procédé de démaquillage d'une zone de la peau sur laquelle est appliqué un cosmétique de maquillage en utilisant une composition de nettoyage comprenant les composants (A) et (B) suivants :

(A) une phase aqueuse contenant de 1 à 25 % en poids, dans la phase aqueuse, d'un composé hydrosoluble (a) qui présente une structure de polyoxypropylène présentant 3 ou plus d'un nombre additionnel d'oxyde de propylène, mais ni un groupe alkyle possédant 4 atomes de carbone ou plus, ni une structure de polyoxyéthylène et
(B) une phase huileuse qui est liquide à 30 °C et contient 50 % en poids ou plus d'une huile (b) présentant un point d'ébullition de 160°C à 300 °C à 1013,25 hPa choisie parmi l'isoparaffine liquide légère et la silicone volatile et comprenant en outre plus de 10 % en poids d'eau par rapport à la totalité de la composition,

dans lequel le rapport pondéral (A)/(B) de la phase aqueuse (A) à la phase huileuse (B) est de 97/3 à 40/60.

10. Procédé selon la revendication 9, dans lequel la composition comprend en outre un tensioactif en quantité de 2 % en poids ou moins par rapport à la totalité de la composition.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5163116 A
- JP 11503461 A
- JP 9048706 A
- JP 62135406 A
- JP 9110638 A
- JP 2104511 A
- JP 9143028 A
- JP 2001302450 A
- JP 2003335627 A
- WO 9503781 A
- JP 9087223 A
- JP 9235301 A